# FASCICULE DE BREVET EUROPEEN

(11) **EP 2 915 501 B1**
(45) Date de publication et mention de la délivrance du brevet: **16.08.2017**
(21) Numéro de dépôt: 15158101.4
(22) Date de dépôt: 06.03.2015
(51) Int. Cl.: A61B 50/20, A61C 19/02, A61B 50/22, A61C 3/04

(54) **Support pour maintenir un outil**
Halterung zur Aufnahme eines Werkzeugs
Storage device for tool

(30) Priorité: 07.03.2014 FR 1451857
(43) Date de publication de la demande: 09.09.2015
(73) Titulaire: Exotec Dentaire, 21800 Sennecey les Dijon (FR)
(72) Inventeur: Blois, Eric, 21800 Crimolois (FR)
(74) Mandataire: Gevers SA

(56) Documents cités:
- FR-A1- 2 968 925
- US-A1- 2007 119 871
- US-A1- 2011 114 522

## Description

### DOMAINE TECHNIQUE

La présente invention concerne un support de rangement d'instruments tels que des forets, par exemple, utilisés par des chirurgiens-dentistes, et plus particulièrement un perfectionnement à un support d'outils.

### ART ANTERIEUR

Dans le domaine du dentaire, il est bien connu d'utiliser plusieurs instruments médicaux pour réaliser un acte déterminé, lesdits instruments tels qu'un foret, un tenon dentaire, des fraises, des alésoirs, etc... sont généralement rangés dans différents boitiers en fonction de leurs dimensions respectives, lesdits instruments présentant généralement une forme cylindrique de section circulaire avec, par exemple, un diamètre maximum qui est inférieur à 3 mm, et une longueur entre deux extrémités, l'une proximale et l'autre distale, inférieure à 50 mm.

Outre le fait qu'un trop grand nombre de boitiers de rangement rend la réalisation de l'acte plus compliqué, il augmente le risque de contamination croisée des instruments. Ainsi, lesdits instruments sont fréquemment stérilisés dans un stérilisateur dans lequel lesdits instruments sont placés en vrac. Après stérilisation, lesdits instruments sont à nouveau rangés dans des boitiers ce qui implique un nombre de manipulations relativement important, les instruments devant être manipulés un par un.

Afin de remédier à ces inconvénients, on a déjà imaginé des supports d'outils permettant de recevoir des outils de longueurs différentes, facilitant la reconnaissance et la saisie desdits outils, et permettant une stérilisation plus aisée.

C'est le cas notamment du brevet américain US 5,829,590, de la demande de brevet international WO2010/0146304 et de la demande de brevet français FR 2 968 925.

Le brevet américain US 5,829,590 décrit un support comprenant une plaque de maintien horizontale et pourvue d'une pluralité d'orifices s'étendant au travers de la plaque de maintien selon une direction verticale, perpendiculaire à la plaque de maintien, chaque orifice étant adapté pour recevoir un outil.

Toutefois, le support connu n'offre pas un maintien satisfaisant de l'outil qui peut s'incliner librement dans l'orifice autour d'un axe perpendiculaire à la direction verticale.

Le document WO 2010/146304 décrit un système de rangement d'instruments comprenant une pièce de base avec un segment plan horizontal et deux segments plans verticaux disposés sous forme d'un U. Des platines perforées sont disposées entre les deux segments plans verticaux et de façon parallèle par rapport au segment plan horizontal de la pièce de base. Les platines perforées sont agencées de façon à ce qu'il existe plusieurs rangées de perforations alignées verticalement. Une rangée de perforations définit une colonne s'étendant de la platine perforée la plus éloignée du segment plan horizontal en direction de celui-ci jusqu'à une platine perforée n'ayant pas de perforations appartenant à la rangée ou, dans le cas où toutes les platines ont des perforations appartenant à la rangée, jusqu'au segment plan horizontal. La colonne a une longueur spécifique, et les perforations de la rangée ont des dimensions spécifiques, afin que la rangée de perforations puisse accueillir un instrument spécifique.

Néanmoins, les plaques de maintien ne sont pas déplaçable par rapport au socle de sorte que ce type de support ne permet de ranger des instruments de longueurs variables.

Le document FR 2 968 925 (le préambule de la revendication 1 est basé sur ce document) décrit un support pour maintenir au moins un outil comprenant une plaque de maintien horizontale et pourvue d'au moins un orifice s'étendant au travers de la plaque de maintien selon une direction verticale perpendiculaire à la plaque de maintien, ledit orifice étant adapté pour recevoir l'outil au voisinage de l'extrémité proximale. Ledit support comprend par ailleurs un socle parallèle à la plaque de maintien, ledit socle présentant une surface d'appui horizontale, en regard de la plaque de maintien et sur laquelle l'extrémité distale de l'outil est destinée à reposer, le socle étant déplaçable par rapport à la plaque de maintien de telle sorte que la surface d'appui soit placée à une distance, mesurée selon la direction verticale, variable par rapport à la plaque de maintien.

Ce type de support présente néanmoins de ne pas permettre une saisie aisée des instruments, notamment des forets.

### EXPOSE DE L'INVENTION

L'un des buts de l'invention est donc de remédier à ces inconvénients en proposant un perfectionnement à un support d'outils de conception simple et peu onéreuse, permettant une saisie aisée des outils mais également du support.

A cet effet et conformément à l'invention, il est proposé un support pour maintenir au moins un outil, notamment un outil de chirurgie, en particulier un outil de chirurgie dentaire, lequel outil présente une longueur entre une extrémité proximale et une extrémité distale, ledit support comportant au moins une plaque de maintien horizontale s'étendant entre deux montants verticaux et pourvue d'au moins un orifice s'étendant au travers de la plaque de maintien, ledit orifice étant adapté pour recevoir l'outil, et un socle s'étendant parallèlement à la plaque de maintien et présentant une surface d'appui horizontale en regard de la plaque de maintien et sur laquelle l'extrémité distale de l'outil est destinée à reposer, ledit socle étant déplaçable par rapport à la plaque de maintien de telle sorte que la surface d'appui soit placée à une distance variable par rapport à la plaque de maintien ; ledit support est remarquable en qu'il comporte un arceau rectangulaire solidaire de l'extrémité supérieure des montants verticaux, ledit arceau s'étendant horizontalement, parallèlement à la plaque de maintien qui s'étend entre les montants verticaux depuis la partie médiane desdits montants, lesdits montants verticaux présentant une épaisseur décroissante depuis la paroi supérieure de la plaque de maintien jusqu'à leurs extrémités supérieures.

De préférence, les montants verticaux présentent une forme sensiblement rectangulaire dont la largeur est inférieure à la largeur de la plaque de maintien, ladite plaque de maintien présentant une largeur inférieure à celle de l'arceau rectangulaire.

Lesdits montants verticaux comportent, en dessous de la plaque de maintien, au moins une paire de lumières, lesdites lumières étant ménagées l'une en regard de l'autre, apte à recevoir des ergots faisant saillies de deux bords opposés du socle.

De manière avantageuse, les ergots de chaque socle présentent une hauteur inférieure à l'épaisseur du socle et sont affleurant à l'une des faces dudit socle permettant ainsi de procurer deux hauteurs différentes pour une même paire de lumières.

De préférence, les montants verticaux comportent trois paires de lumières ménagées l'une en regard de l'autre.

Lesdites paires de lumières sont régulièrement espacées entre la paroi inférieure de la plaque de maintien et les extrémités inférieures des montants verticaux.

Par ailleurs, le socle présente une forme sensiblement rectangulaire dont la longueur est sensiblement égale à la distance séparant les deux montants verticaux et dont la largeur est sensiblement égale à la largeur desdits montants.

Lesdites lumières présentent une forme rectangulaire et les ergots du socle présentent une forme sensiblement parallélépipédique.

De plus, la plaque de maintien comporte sur sa paroi inférieure des alésages s'étendant coaxialement aux orifices de ladite plaque de maintien, lesdits alésages procurant un meilleur maintien vertical des outils.

De manière avantageuse, le support comporte également deux pattes verticales, formant des organes de préhension, s'étendant au-dessus de l'arceau rectangulaire dans le prolongement des montants verticaux.

Accessoirement, le support comporte un premier couvercle comprenant une coiffe sensiblement parallélépipédique dont le bord inférieur est apte à prendre appui sur l'arceau rectangulaire et deux parois opposées aptes à s'étendre entre ledit arceau rectangulaire et les montants verticaux, jusqu'aux extrémités inférieures desdits montants verticaux.

Il comporte également un second couvercle comprenant une coiffe sensiblement parallélépipédique dont le bord inférieur est apte à prendre appui sur l'arceau rectangulaire et dont les bords longitudinaux sont munis de pattes de fixation aptes à prendre appui sur le bord intérieur de l'arceau rectangulaire pour assurer le maintien dudit couvercle.

De préférence, les parois longitudinales de la coiffe du second couvercle présentent un creux afin de faciliter la préhension dudit couvercle.

### DESCRIPTION SOMMAIRE DES FIGURES

D'autres avantages et caractéristiques ressortiront mieux de la description qui va suivre de plusieurs variantes d'exécution, données à titre d'exemples non limitatifs, du support d'outil conforme à l'invention, en référence aux dessins annexés sur lesquels :
- la figure 1 est une vue en perspective éclatée d'un ensemble comprenant le support d'outil suivant l'invention,
- la figure 2 est une vue en perspective éclatée du support d'outils suivant l'invention,
- la figure 3 est une vue de côté du support d'outils suivant l'invention,
- les figures 4A et 4B sont des vues de côté schématiques du socle du support d'outil d'invention côté endroit et respectivement envers,
- la figure 5 est une vue en élévation du support d'outils suivant l'invention,
- la figure 6 est une vue de côté du support d'outil suivant l'invention,
- la figure 7 est une de face d'un couvercle du support d'outil suivant l'invention,
- la figure 8 est une vue de dessus du couvercle du support d'outil suivant l'invention représenté sur la figure 7,
- la figure 9 est une vue de côté du couvercle du support d'outil suivant l'invention représenté sur les figures 7 et 8,
- la figure 10 est une vue de dessous d'un autre couvercle du support d'outil suivant l'invention,
- la figure 11 est une vue de côté du couvercle du support d'outil suivant l'invention représenté sur la figure 10,
- la figure 12 est une vue en perspective de dessous des bases de deux boitiers aptes à recevoir des supports d'outil suivant l'invention,
- la figure 13 est une vue en perspective de dessus des bases de deux boitiers aptes à recevoir des supports d'outil suivant l'invention.

### DESCRIPTION DETAILLEE DE L'INVENTION

Dans la suite de la description du support d'outil suivant l'invention, les mêmes références numériques désignent les mêmes éléments. Par ailleurs, les différentes vues ne sont pas nécessairement tracées à l'échelle. Le support suivant l'invention est particulièrement destiné pour maintenir un ou plusieurs outils de chirurgie dentaire, tels que des tenons, des clavettes, des forets, des fraises, des alésoirs ou d'autres outils. Dans l'application considérée, les outils, en matériau biocompatible, ont une forme cylindrique, généralement de section circulaire, selon un axe central. Ils s'étendent entre une extrémité proximale adaptée notamment pour en permettre la saisie et la solidarisation à un autre organe, et une extrémité distale, en forme de pointe, adaptée pour pénétrer, et éventuellement s'ancrer, dans les tissus. En particulier, dans le domaine de la chirurgie dentaire, les tenons et les clavettes qui constituent des éléments de fixation d'une prothèse dentaire ont leur extrémité proximale conformée pour permettre leur solidarisation à la prothèse. Les forets qui constituent des éléments de forage ont leur extrémité proximale conformée pour permettre leur solidarisation à un instrument de forage, éventuellement motorisé. De tels outils présentent, par ailleurs, généralement des dimensions réduites et, en particulier, un diamètre maximum qui est inférieur à 3 mm, et une longueur entre les extrémités proximale et distale inférieure à 50 mm. Bien que décrite en relation avec des outils de chirurgie dentaire, l'invention s'applique également au maintien d'éléments de fixation et de forage et plus généralement d'outils utilisés dans d'autres domaines de la chirurgie ou d'outils de domaines autre que la chirurgie, lesquels outils pouvant alors présenter des formes et des dimensions différentes de celles décrites précédemment.

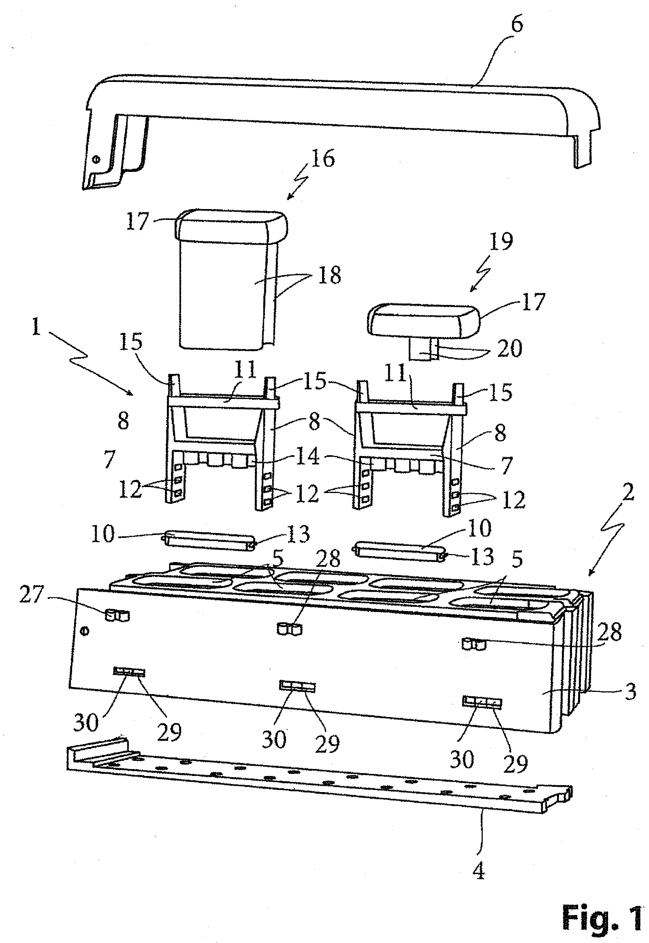

En référence à la figure 1, le support (1) suivant l'invention peut être utilisé pour réaliser un ensemble (2) modulable d'un ou plusieurs types d'outils. Ledit ensemble (2) comporte une base (3) dont l'extrémité inférieure est ouverte et apte à être fermée par une plaque dite d'obturation (4) et dont la paroi supérieure comporte des ouvertures (5) apte à recevoir des supports (1) comme il sera détaillé plus loin. Par ailleurs, l'ensemble comporte également un capot (6) articulé à la base (3) et apte à coiffer les supports (1) positionnés dans les ouvertures (5) de ladite base.

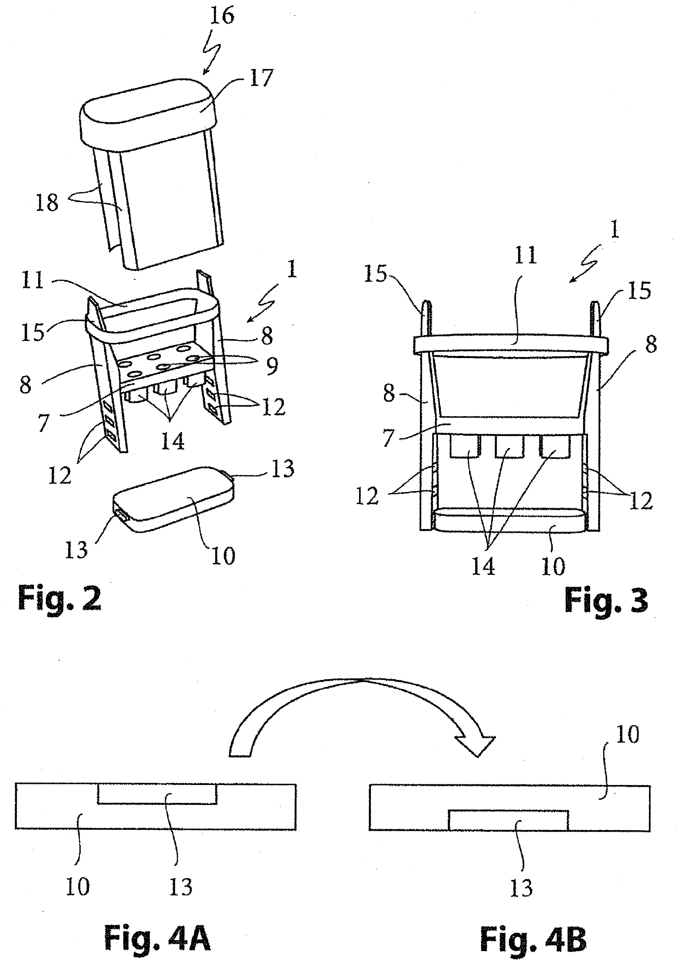

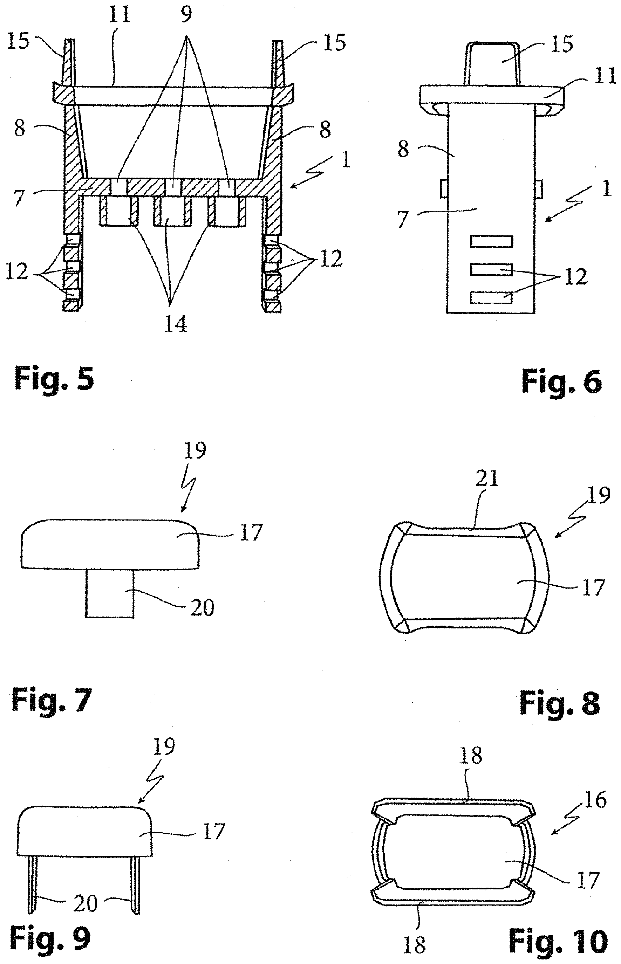

En référence aux figures 1 à 6, ledit support suivant l'invention comporte une plaque de maintien (7) horizontale s'étendant entre deux montants verticaux (8) et pourvue d'une pluralité d'orifices (9) s'étendant au travers de la plaque de maintien (7), lesdits orifices (9) étant adaptés pour recevoir des outils tels qu'un foret, un tenon dentaire, un alésoir, etc... et un socle (10) s'étendant parallèlement à la plaque de maintien (7) et présentant une surface d'appui horizontale en regard de la plaque de maintien (7) et sur laquelle l'extrémité distale de l'outil est destinée à reposer, ledit socle (10) étant déplaçable par rapport à la plaque de maintien (7) de telle sorte que la surface d'appui soit placée à une distance variable par rapport à la plaque de maintien (7). Dans cet exemple particulier de réalisation, la plaque de maintien (7) comporte deux rangées de trois orifices (9) ; toutefois, il va de soi que ladite plaque de maintien (7) pourra comporter un nombre quelconque d'orifices (9) sans pour autant sortir du cadre de l'invention.

Par ailleurs, le support (1) comporte un arceau rectangulaire (11) solidaire de l'extrémité supérieure des montants verticaux (8), ledit arceau (11) s'étendant horizontalement, parallèlement à la plaque de maintien (7) qui s'étend entre les montants verticaux (8) depuis la partie médiane desdits montants (8), lesdits montants verticaux (8) présentant une épaisseur décroissante depuis la paroi supérieure de la plaque de maintien (7) jusqu'à leurs extrémités supérieures. De cette manière, la paroi intérieure des montants verticaux (8) s'évase vers l'extérieur depuis la plaque de maintien (8) permettant une saisie plus aisée des outils.

Les montants verticaux (8) présentent une forme sensiblement rectangulaire dont la largeur est inférieure à la largeur de la plaque de maintien (7), ladite plaque de maintien (7) présentant une largeur inférieure à celle de l'arceau rectangulaire (11). Ainsi, les bords longitudinaux de la plaque de maintien font légèrement saillie des montants verticaux (8). Lesdits montants verticaux (8) comportent, en dessous de la plaque de maintien (7), au moins une paire de lumières (12), lesdites lumières (12) étant ménagées l'une en regard de l'autre. Dans cet exemple particulier de réalisation, les montants verticaux (8) comportent trois paires de lumières (12) ménagées l'une en regard de l'autre, lesdites paires de lumières (12) étant régulièrement espacées entre la paroi inférieure de la plaque de maintien (7) et les extrémités inférieures des montants verticaux (8). Toutefois, il est bien évident que les montants pourront comporter un nombre quelconque de lumières (12) régulièrement espacées ou non sans pour autant sortir du cadre de l'invention. Lesdites lumières (12) sont aptes à recevoir des ergots (13) faisant saillies de deux bords opposés du socle (10). Ledit socle (10) présente une forme sensiblement rectangulaire dont la longueur est sensiblement égale à la distance séparant les deux montants verticaux (8) et dont la largeur est sensiblement égale à la largeur desdits montants (8). Lesdites lumières (12) présentent une forme rectangulaire et les ergots (13) du socle (10) présentent une forme sensiblement parallélépipédique. De manière avantageuse, les ergots (13) de chaque socle (10) présentent une hauteur inférieure à l'épaisseur du socle (10) et sont affleurant à l'une des faces dudit socle (10). De cette manière, à partir d'un même socle (10) il est possible d'obtenir deux hauteurs différentes pour une même paire de lumières (12) en présentant l'une ou l'autre des faces du socle (10) au droit de la plaque de maintien. Ainsi, à partir de trois paires de lumières (12), il est possible d'obtenir six hauteurs différentes, ce qui permet de réduire le nombre de pièces et donc le coût du support.

Il va de soi que les ergots (13) du socle (10) pourront présenter une forme quelconque et que les lumières (12) pourront présenter une forme complémentaire auxdits ergots (13) sans pour autant sortir du cadre de l'invention.

Par ailleurs, afin d'assurer un meilleur maintien vertical des outils, ladite plaque de maintien (7) comporte sur sa paroi inférieure des alésages (14) cylindriques s'étendant coaxialement aux orifices (9) de ladite plaque de maintien (7).

Bien entendu, le nombre, l'agencement, la forme et les dimensions des orifices (9) et des alésages (14) pourraient être différents de ceux décrits précédemment pour s'adapter à l'application considérée et aux outils devant être maintenus.

Accessoirement, le support suivant l'invention comporte deux pattes verticales (15), formant des organes de préhension, s'étendant au-dessus de l'arceau rectangulaire (11) dans le prolongement des montants verticaux (8). Ces pattes verticales (15) permettent de saisir le support entre le pouce et l'index sans toucher les outils positionnés sur ledit support évitant ainsi toute contamination.

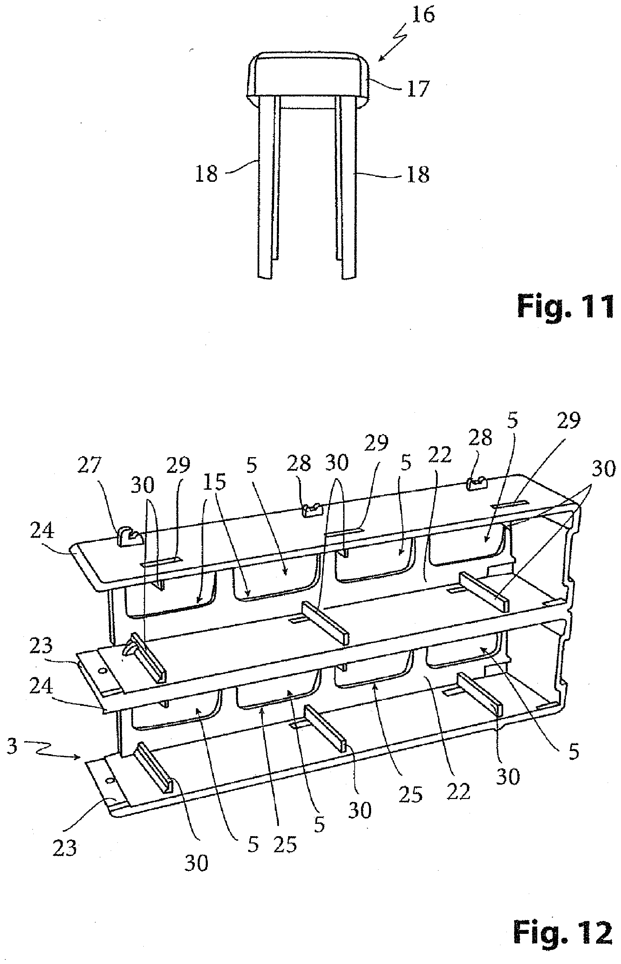

Par ailleurs, en référence aux figures 1, 10 et 11, le support comporte un premier couvercle (16) comprenant une coiffe (17) sensiblement parallélépipédique dont le bord inférieur est apte à prendre appui sur l'arceau rectangulaire (11) et deux parois opposées (18) aptes à s'étendre entre ledit arceau rectangulaire (11) et les montants verticaux (8), jusqu'aux extrémités inférieures desdits montants verticaux (8).

De plus, en référence aux figures 1 et 7 à 9, le support comporte un second couvercle (19) comprenant une coiffe (17) sensiblement parallélépipédique dont le bord inférieur est apte à prendre appui sur l'arceau rectangulaire (11) et dont les bords longitudinaux sont munis de pattes de fixation (20) aptes à prendre appui sur le bord intérieur de l'arceau rectangulaire (11) pour assurer le maintien dudit couvercle (19). Les parois longitudinales de la coiffe (17) du second couvercle (19) présentent avantageusement un creux (21) afin de faciliter la préhension dudit couvercle (19). Ce second couvercle permet de préserver les outils

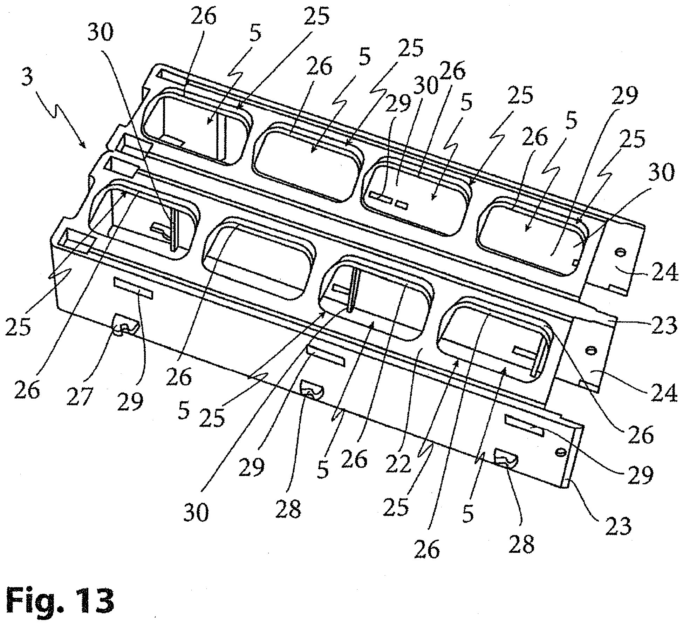

Accessoirement, en référence aux figures 1, 12 et 13, la base (3) représentée sur les comporte une âme (22) horizontale et deux ailes latérales (23,24) s'étendant depuis l'âme (22) perpendiculairement à celle-ci, en regard l'une de l'autre. L'âme (22) et les ailes latérales (23,24) définissent un espace intérieur. La base (3) peut également être complétée avec une plaque d'obturation (4) solidarisée de manière appropriée aux ailes latérales (23,24), à l'opposé de l'âme (22), pour fermer l'espace intérieur de la base (3). L'âme (22) présente une surface intérieure tournée vers l'espace intérieur et une surface extérieure opposée. L'âme (22) est pourvue d'ouvertures (5) pour faire communiquer l'espace intérieur de la base (3) avec l'extérieur. Lesdites ouvertures (5) sont adaptées pour recevoir chacune l'un des supports 1. Chacune des ouvertures (5) est délimitée par un épaulement périphérique (25) présentant une épaisseur et un contour correspondant sensiblement à ceux de l'arceau rectangulaire (11). Sur la surface intérieure de l'âme (22), chacune des ouvertures (5) est, par ailleurs, pourvue d'une bordure (26) saillante par rapport au bord périphérique. Ainsi une partie inférieure de chacun des supports 1, située sous l'arceau rectangulaire (11), peut être introduite dans l'espace intérieur de la base (3) par l'ouverture (5) correspondante, jusqu'à ce que l'arceau rectangulaire (11) prenne appui sur la bordure (26). La surface supérieure de l'arceau rectangulaire (11) affleure la surface extérieure de l'âme (22) de sorte à faciliter la préhension des outils. Les pattes (15) du support (1) permettent de saisir ledit support (1) pour le placer sur la base (3) ou l'en extraire.

De plus, les ailes latérales (23,24) présentent également chacune une surface intérieure tournée vers l'espace intérieur et une surface extérieure opposée. Lesdites parois latérales (23,24) sont pourvues d'organes de solidarisation pour permettre la solidarisation des bases (3) de deux boîtiers identiques. En particulier, les organes de solidarisation de chacune de parois latérales (23,24) comprennent un crochet (27) et deux butées (28) alignés selon la direction longitudinale, saillants selon la direction transversale sur la surface extérieure, des ouvertures (29) alignées selon la direction longitudinale. Le crochet (27) et les butées (28) de l'une (23), première, des ailes latérales (23,24) sont placés à proximité d'un bord inférieur de la première paroi latérale (23), les ouvertures (29) étant placées à proximité d'un bord supérieur de la première paroi latérale (23). Inversement, le crochet (27) et les butées (28) de l'autre (24), deuxième, des ailes latérales sont placés à proximité d'un bord supérieur de la deuxième paroi latérale (24), les ouvertures (29) étant placées à proximité d'un bord inférieur de la deuxième paroi latérale (24). De cette manière, le crochet (27) et les butées (28) de la première paroi latérale (23) de l'une des bases (3) peuvent s'engager dans les ouvertures (29) de la deuxième paroi latérale (24) de l'autre base (3). Pour améliorer la solidarisation, la surface intérieure des parois latérales (23,24) peut être pourvue de tiges (30) s'étendant selon la direction verticale et agencée pour renforcer la tenue et le contact du crochet (27) et des butées (28) dans les ouvertures (29).

On observera que le premier couvercle (16) et/ou le second couvercle (19) et/ou le capot (6) pourront avantageusement être obtenus dans une matière plastique transparente ou translucide afin de permettre une visualisation des outils positionnés sur les supports.

De plus, le support (1), et plus particulièrement la plaque de maintien (7) et les montants verticaux (8), pourront être obtenus dans une matière plastique teintée dans la masse de telle sorte que lesdits supports (2) présentent la même couleur que la bague colorée des outils placés sur ledit support (2). La bague colorée des fraises, par exemple, est une indication standard selon la norme ISO du type de grosseur des grains de diamant de la fraise. Ainsi, un praticien peut repérer plus simplement et plus rapidement l'outil dont il a besoin. Lesdits plastiques sont, de préférence, des plastiques stérilisables bien connus de l'homme du métier.

Enfin, il est bien évident que les exemples que l'on vient de donner ne sont que des illustrations particulières et en aucun cas limitatif quant au domaine d'application de l'invention.

## Revendications

1. Support pour maintenir au moins un outil, notamment un outil de chirurgie, en particulier un outil de chirurgie dentaire, lequel outil présente une longueur entre une extrémité proximale et une extrémité distale, ledit support comportant au moins une plaque de maintien (7) horizontale s'étendant entre deux montants verticaux (8) et pourvue d'au moins un orifice (9) s'étendant au travers de la plaque de maintien (7), ledit orifice (9) étant adapté pour recevoir l'outil, et un socle (10) s'étendant parallèlement à la plaque de maintien (7) et présentant une surface d'appui horizontale en regard de la plaque de maintien (7) et sur laquelle l'extrémité distale de l'outil est destinée à reposer, ledit socle (10) étant déplaçable par rapport à la plaque de maintien (7) de telle sorte que la surface d'appui soit placée à une distance variable par rapport à la plaque de maintien *(7), caractérisé* en qu'il comporte un arceau rectangulaire (11) solidaire de l'extrémité supérieure des montants verticaux (8), ledit arceau (11) s'étendant horizontalement, parallèlement à la plaque de maintien (7) qui s'étend entre les montants verticaux (8) depuis la partie médiane desdits montants (8), lesdits montants verticaux (8) présentant une épaisseur décroissante depuis la paroi supérieure de la plaque de maintien (7) jusqu'à leurs extrémités supérieures.

2. Support suivant la revendication précédente ***caractérisé* en ce que** les montants verticaux (8) présentent une forme sensiblement rectangulaire dont la largeur est inférieure à la largeur de la plaque de maintien (7), ladite plaque de maintien (7) présentant une largeur inférieure à celle de l'arceau rectangulaire (11).

3. Support suivant l'une quelconque des revendications 1 ou 2 ***caractérisé* en ce que** les montants verticaux (8) comportent, en dessous de la plaque de maintien (7), au moins une paire de lumières (12), lesdites lumières (12) étant ménagées l'une en regard de l'autre, apte à recevoir des ergots (13) faisant saillies de deux bords opposés du socle (10).

4. Support suivant la revendication 3 ***caractérisé* en ce que** les ergots (13) de chaque socle (10) présentent une hauteur inférieure à l'épaisseur du socle (10) et sont affleurant à l'une des faces dudit socle (10).

5. Support suivant l'une quelconque des revendication 3 ou 4 ***caractérisé* en ce que** les montants verticaux (8) comportent trois paires de lumières (12) ménagées l'une en regard de l'autre.

6. Support suivant l'une quelconque des revendications 3 à 5 ***caractérisé* en ce que** les paires de lumières (12) sont régulièrement espacées entre la paroi inférieure de la plaque de maintien (7) et les extrémités inférieures des montants verticaux (8).

7. Support suivant l'une quelconque des revendications 3 à 6 ***caractérisé* en ce que** le socle (10) présente une forme sensiblement rectangulaire dont la longueur est sensiblement égale à la distance séparant les deux montants verticaux (8) et dont la largeur est sensiblement égale à la largeur desdits montants (8).

8. Support suivant l'une quelconque des revendications 3 à 7 ***caractérisé* en ce que** lesdites lumières (12) présentent une forme rectangulaire et les ergots (13) du socle (10) présentent une forme sensiblement parallélépipédique.

9. Support suivant l'une quelconque des revendications 1 à 8 ***caractérisé* en ce que** la plaque de maintien (7) comporte sur sa paroi inférieure des alésages (14) s'étendant coaxialement aux orifices (9) de ladite plaque de maintien (7).

10. Support suivant l'une quelconque des revendications 1 à 9 ***caractérisé* en ce qu'**il comporte deux pattes verticales (15), formant des organes de préhension, s'étendant au-dessus de l'arceau rectangulaire (11) dans le prolongement des montants verticaux (8).

11. Support suivant l'une quelconque des revendications 1 à 10 ***caractérisé* en ce qu'**il comporte un premier couvercle (16) comprenant une coiffe (17) sensiblement parallélépipédique dont le bord inférieur est apte à prendre appui sur l'arceau rectangulaire (11) et deux parois opposées (18) aptes à s'étendre entre ledit arceau rectangulaire (11) et les montants verticaux (8), jusqu'aux extrémités inférieures desdits montants verticaux (8).

12. Support suivant l'une quelconque des revendications 1 à 10 ***caractérisé* en ce qu'**il comporte un second couvercle (19) comprenant une coiffe (17) sensiblement parallélépipédique dont le bord inférieur est apte à prendre appui sur l'arceau rectangulaire (11) et dont les bords longitudinaux sont munis de pattes de fixation (20) aptes à prendre appui sur le bord intérieur de l'arceau rectangulaire (11) pour assurer le maintien dudit couvercle (19).

13. Support suivant la revendication 12 ***caractérisé* en ce que** les parois longitudinales de la coiffe (17) du second couvercle (19) présentent un creux (21) afin de faciliter la préhension dudit couvercle (19).

## Patentansprüche

1. Träger, um mindestens ein Werkzeug zu halten, insbesondere ein chirurgisches Werkzeug, insbesondere ein zahnchirurgisches Werkzeug, wobei das Werkzeug eine Länge zwischen einem proximalen Ende und einem distalen Ende aufweist, wobei der Träger mindestens eine horizontale Halteplatte (7) umfasst, die sich zwischen zwei vertikalen Säulen (8) erstreckt, und versehen mit mindestens einer Öffnung (9), die sich quer über die Halteplatte (7) erstreckt, wobei die Öffnung (9) ausgelegt ist, um das Werkzeug aufzunehmen, und einen Sockel (10), der sich parallel zur Halteplatte (7) erstreckt und eine horizontale Auflagefläche gegenüber der Halteplatte (7) aufweist, und auf der das distale Ende des Werkzeugs lagern soll, wobei der Sockel (10) mit Bezug auf die Halteplatte (7) derart verschoben werden kann, dass die Auflagefläche in einer variablen Distanz mit Bezug auf die Halteplatte (7) angeordnet ist, **dadurch gekennzeichnet, dass** er einen rechtwinkligen Bogen (11) umfasst, der mit dem oberen Ende der vertikalen Säulen (8) einstückig ist, wobei sich der Bogen (11) horizontal erstreckt, parallel zur Halteplatte (7), die sich zwischen den vertikalen Säulen (8) vom mittleren Teil der Säulen (8) erstreckt, wobei die vertikalen Säulen (8) eine abnehmende Dicke von der oberen Wand der Halteplatte (7) bis zu ihren oberen Enden aufweisen.

2. Träger nach dem vorhergehenden Anspruch, **dadurch gekennzeichnet, dass** die vertikalen Säulen (8) eine im Wesentlichen rechtwinklige Form aufweisen, deren Breite geringer als die Breite der Halteplatte (7) ist, wobei die Halteplatte (7) eine Breite aufweist, die geringer als diejenige des rechtwinkligen Bogens (11) ist.

3. Träger nach einem beliebigen der Ansprüche 1 oder 2, **dadurch gekennzeichnet, dass** die vertikalen Säulen (8) unter der Halteplatte (7) mindestens ein Paar Löcher (12) umfassen, wobei die Löcher (12) einander gegenüber liegend angebracht sind, dazu ausgelegt, Zapfen (13) aufzunehmen, die von zwei einander gegenüber liegenden Rändern des Sockels (10) hervorspringen.

4. Träger nach Anspruch 3, **dadurch gekennzeichnet, dass** die Zapfen (13) jedes Sockels (10) eine Höhe aufweisen, die geringer als die Dicke des Sockels (10) ist und mit einem der Seiten des Sockels (10) bündig sind.

5. Träger nach einem beliebigen der Ansprüche 3 oder 4, **dadurch gekennzeichnet, dass** die vertikalen Säulen (8) drei Paar Löcher (12) umfassen, die einander gegenüber liegend angebracht sind.

6. Träger nach einem beliebigen der Ansprüche 3 bis 5, **dadurch gekennzeichnet, dass** die Löcher (12) regelmäßig zwischen der unteren Wand der Halteplatte (7) und den unteren Enden der vertikalen Säulen (8) verteilt sind.

7. Träger nach einem beliebigen der Ansprüche 3 bis 6, **dadurch gekennzeichnet, dass** der Sockel (10) eine im Wesentlichen rechtwinklige Form aufweist, deren Länge im Wesentlichen gleich dem Abstand ist, der die zwei vertikalen Säulen (8) trennt, und deren Breite im Wesentlichen gleich der Breite der Säulen (8) ist.

8. Träger nach einem beliebigen der Ansprüche 3 bis 7, **dadurch gekennzeichnet, dass** die Löcher (12) eine rechtwinklige Form aufweisen und die Zapfen (13) des Sockels (10) eine im Wesentlichen parallelepipede Form aufweisen.

9. Träger nach einem beliebigen der Ansprüche 1 bis 8, **dadurch gekennzeichnet, dass** die Halteplatte (7) auf ihrer unteren Wand Bohrungen (14) umfasst, die sich koaxial zu den Öffnungen (9) der Halteplatte (7) erstrecken.

10. Träger nach einem beliebigen der Ansprüche 1 bis 9, **dadurch gekennzeichnet, dass** er zwei vertikale Füße (15) umfasst, die Greiforgane bilden, die sich über dem rechtwinkligen Bogen (11) in der Verlängerung der vertikalen Säulen (8) erstrecken.

11. Träger nach einem beliebigen der Ansprüche 1 bis 10, **dadurch gekennzeichnet, dass** er einen ersten Deckel (16) umfasst, der eine im Wesentlichen parallelepipede Abdeckung (17) aufweist, deren unterer Rand ausgelegt ist, um auf dem rechtwinkligen Bogen (11) aufzuliegen, und zwei gegenüber liegende Wände (18), die ausgelegt sind, um sich zwischen dem rechtwinkligen Bogen (11) und den vertikalen Säulen (8) bis zu den unteren Enden der vertikalen Säulen (8) zu erstrecken.

12. Träger nach einem beliebigen der Ansprüche 1 bis 10, **dadurch gekennzeichnet, dass** er einen zweiten Deckel (19) umfasst, der eine im Wesentlichen parallelepipede Abdeckung (17) umfasst, deren unterer Rand Längsränder mit Befestigungsfüßen (20) ausgestattet sind, die ausgelegt sind, um auf dem Innenrand des rechtwinkligen Bogens (11) aufzuliegen, um den Halt des Deckels (19) sicherzustellen.

13. Träger nach Anspruch 12, **dadurch gekennzeichnet, dass** die Längswände der Abdeckung (17) des zweiten Deckels (19) einen Hohlraum (21) darstellen, um das Greifen Deckels (19) zu erleichtern.

## Claims

1. A support for holding at least one tool, in particular a surgical tool, particularly a dental surgical tool, having a length between a proximal end and a distal end, said support including at least one horizontal maintaining plate (7) extending between two vertical posts (8) and provided with at least one orifice (9) extending through the maintaining plate (7), said orifice (9) being suitable for receiving the tool, and the base (10) extending parallel to the maintaining plate (7) and having a horizontal bearing surface across from the maintaining plate (7) and on which the distal end of the tool is intended to rest, said base (10) being movable relative to the maintaining plate (7) such that the bearing surface is placed a variable distance relative to the maintaining plate (7), ***characterized* in that** it includes a rectangular cradle (11) secured to the upper end of the vertical posts (8), said cradle (11) extending horizontally, parallel to the maintaining plate (7) which extends between the vertical posts (8) from the median part of said posts (8), said vertical posts (8) having a decrease in thickness from the upper wall of the maintaining plate (7) to their upper ends.

2. The support according to the preceding claim, ***characterized* in that** the vertical posts (8) have a substantially rectangular shape, the width of which is smaller than the width of the maintaining plate (7), said maintaining plate (7) having a width smaller than that of the rectangular cradle (11).

3. The support according to any one of claims 1 or 2, ***characterized* in that** the vertical posts (8) includes, below the maintaining plate (7), at least one pair of apertures (12), said apertures (12) being arranged across from one another, able to receive snugs (13) protruding from two opposite edges of the base (10).

4. The support according to claim 3, ***characterized* in that** the snugs (13) of each base (10) have a height smaller than the thickness of the base (10) and/or flush with one of the faces of said base (10).

5. The support according to any one of claims 3 or 4, **characterized in that** the vertical posts (8) includes three pairs of apertures (12) arranged across from one another.

6. The support according to any one of claims 3 to 5, ***characterized* in that** the pairs of apertures (12) are regularly spaced between the lower wall of the maintaining plate (7) and the lower ends of the vertical posts (8).

7. The support according to any one of claims 3 to 6, ***characterized* in that** the base (10) has a substantially rectangular shape, the length of which is substantially equal to the distance separating the two vertical posts (8) and the width of which is substantially equal to the width of said posts (8).

8. The support according to any one of claims 3 to 7, ***characterized* in that** said apertures (12) have a rectangular shape and the snugs (13) of the base (10) have a substantially parallelepiped shape.

9. The support according to any one of claims that were once 8, ***characterized* in that** the maintaining plate (7) includes, on its lower face, borders (14) extending coaxially to the orifices (9) of said maintaining plate (7).

10. The support according to any one of claims 1 and 9, ***characterized* in that** it includes two vertical tabs (15), forming gripping members, extending above the rectangular cradle (11) in the extension of the vertical posts (8).

11. The support according to any one of claims 1 to 10, ***characterized* in that** it includes a first cover (16) comprising a substantially parallelepiped cap (17) where of the lower edge is able to bear on the rectangular cradle (11) and two opposite walls (18) able to extend between said rectangular cradle (11) and the vertical posts (8), up to the lower ends of said vertical posts (8).

12. The support according to any one of claims 1 to 10, ***characterized* in that** it includes a second cover (19) comprising a substantially parallelepiped cap (17) where of the lower edge is able to bear on the rectangle cradle (11) and the longitudinal edges of which are provided with fastening tabs (20) able to bear on the inner edge of the rectangular cradle (11) to maintain said cover (19).

13. The support according to claim 12, ***characterized* in that** the longitudinal walls of the cap (17) of the second cover (19) have a recess (21) in order to facilitate the gripping of said cover (19).
